# EUROPEAN PATENT APPLICATION

(11) **EP 3 207 899 A1**
(43) Date of publication of application: **23.08.2017**
(21) Application number: 16425012.8
(22) Date of filing: 16.02.2016
(51) Int. Cl.: A61F 2/08, A61B 17/68, A61B 17/86, A61B 17/88

(54) **ARTIFICIAL CRUCIATE LIGAMENT COMPRISING BUSHINGS AND DEVICE USED FOR PLACING SAID BUSHINGS IN POSITION**

(71) Applicant: Argotech Srl, 60033 Chiaravalle (AN) (IT)
(72) Inventor: Omini, Luca, I-60020 Polverigi (AN) (IT)
(74) Representative: Baldi, Claudio

(57) **Abstract**

An artificial cruciate ligament (C) comprises: a tibial screw (1), a femoral screw (2), a pin (4) and a threaded bolt (5) inserted in the femoral screw (2), thread-like connection means (L) inserted in the tibial screw (1) and in the femoral screw (2), spring bushings (3) intended to be disposed inside said threaded holes, in such manner that the tibial screw (1) and the femoral screw (2) are interfaced with the spring bushings (3); the spring bushings (3) are inserted in the threaded holes by means of a device (100) comprising: a mounting key (A) that is engaged inside the spring bushing (3), a pre-winder (P) configured in such manner to house the mounting key (A) and a stopper (13) to lock the rotation of the mounting key (A) in anticlockwise direction.

## Description

The present patent application for industrial invention relates to an artificial cruciate ligament comprising one or more bushings and to a device used for placing said bushings in position in order to reconstruct the knee joint.

The knee joint has a first and a second ligament, which are defined as cruciate ligaments and are disposed on the sagittal plane between tibia and femur. The first ligament has a caudio-cranial direction and the second ligament has a craniocaudal direction.

The function of the cruciate ligaments is to limit the front and/or rear translation movement of the tibia with respect to the femur; in other words, they ensure the sagittal and rotational stability of the knee joint.

The cruciate ligaments undergo high mechanical stress that can cause a total or partial ligament injury over time.

When this happens, a surgical operation is recommended in order to implant an artificial cruciate ligament to replace the injured cruciate ligament.

Normally, the artificial cruciate ligament comprises a threaded tibial screw disposed in the tibia, a threaded femoral screw disposed in the femur and thread-like connecting means that are connected to the tibial screw and to the femoral screw. The thread-like connecting means can be artificial means made of synthetic tissues, or natural means made of a tendon.

As it is known, the method used for placing the artificial cruciate ligament in position provides for drilling a threaded hole in the tibia and a threaded hole in the femur. Each threaded hole comprises a thread.

Successively, the tibial screw is screwed in the threaded hole obtained on the tibia and the femoral screw is screwed in the threaded hole obtained on the femur. The threaded screws inserted in the threaded holes. act as fixing point for the thread-like connecting means.

The main drawback of such a method is related to the fact that the threaded screws that are directly inserted in the threaded holes may impair the functionality of the knee joint over time due to the fact that the bone that holds the threaded screws is delicate and pliable.

With the passing of time, the thread of the threaded holes may break and the diameter of the threaded holes may increase because of the bone reabsorption in correspondence of the threaded holes. The expression "bone reabsorption" indicates the demolition of bone areas around the threaded holes.

In this way, the tibial screw and the femoral screw may lose their grip in the bone.

Obviously, as soon as the screw loses its firm grip in the bone, every stress becomes very dangerous, with the risk of accelerating the bone reabsorption and making the screw come out from the threaded hole.

The risk of bone reabsorption increases in case of stress caused by physical effort and/or activity because the stress is discharged only on the first two/three turns of the thread and is not distributed on the entire thread.

In other words, the bone tissue has a low mechanical resistance in correspondence of said threaded holes, and this may invalidate the placing in position of the artificial cruciate ligament.

The purpose of the present invention is to provide an artificial cruciate ligament that is more reliable than the known types of artificial cruciate ligaments that are currently used on the market.

Another purpose of the present invention is to devise an artificial cruciate that is easy and fast to implant in the bone.

An additional purpose of the present invention is to provide a device used for placing said artificial cruciate ligament in position, which is simple to use, reliable and efficient.

These purposes have been achieved with the artificial cruciate ligament of the invention, the main features of which are disclosed in the first claim.

An artificial cruciate ligament according to the invention comprises:
- a tibial screw intended to be disposed inside a threaded hole obtained on a tibia and comprising a through axial cavity; said tibial screw comprising a first shaped end section intended to cooperate with a tool to rotate the tibial screw;
- a femoral screw intended to be disposed inside a threaded hole obtained on a femur; said femoral screw comprising a first closed end section and a second end section whereon an axial threaded opening is obtained; said femoral screw comprising a longitudinal blind hole that can be accessed through the axial threaded opening on one side and is closed by said first closed end section on the other side; said femoral screw comprising a through radial hole in proximal position to said first closed end section; said second end section being shaped and intended to cooperate with a tool to rotate the femoral screw;
- a pin slidingly inserted in the cavity of the femoral screw;
- a threaded bolt disposed in the cavity of the femoral screw, having a first end stopped against the pin and a second shaped end intended to cooperate with a tool to rotate the threaded bolt;
- thread-like connection means inserted in the axial cavity of the tibial screw and in the through radial hole of the femoral screw; said thread-like connecting means being interposed and compressed between the pin and the first closed end section of the femoral screw.

The peculiarity of the artificial cruciate ligament according to the present invention consists in the fact that it comprises two spring bushings intended to be respectively disposed in the threaded hole of the tibia and in the threaded hole of the femur.

The tibial screw and the femoral screw are interfaced with the spring bushings. The spring bushings have an internal diameter and an external diameter and comprise turns with a pitch.

In order to insert the spring bushings in the threaded holes obtained on the tibia and on the femur, a device for placing in position the spring bushings of the artificial cruciate ligament has been devised.

The device comprises a mounting key configured in such manner to be engaged inside the spring bushing. The mounting key comprises:
- a stem with circular section comprising a threaded end section configured in such manner to be inserted in the turns of the spring bushing; said threaded end section comprising an axial notch and an oscillating blade that is disposed inside said axial notch; said oscillating blade ending with a striker configured in such manner to be attached to the fixing and driving notch of the spring bushing;
- a screwing handle intended to be held by a user;
- a bar connecting said screwing handle to the stem; said bar comprising ribs and grooves.

Moreover, the device comprises a pre-winder comprising:
- a tubular rod provided with an axial cavity configured in such manner to house the mounting key; said axial cavity comprising an inlet to let the mounting key enter the axial cavity and an outlet to let the mounting key come out from the axial cavity; said inlet having a higher diameter than the external diameter of the spring bushing; said outlet having a slightly lower diameter than the external diameter of the spring bushing; said outlet being threaded with the same pitch as the turns of the spring bushings;
- a holding collar comprising a central hole wherein the tubular rod is inserted.

The device comprises a stopper that cooperates with the bar of the mounting key, locking the rotation of the mounting key in anticlockwise direction.

For purpose of clarity, the description of the artificial cruciate ligament and of the device of the invention continues with reference to the attached drawings, which are intended for purposes of illustration only, and not in a limiting sense, wherein:
- Figs. 1 and 1A are respectively a longitudinal section and a rear view of a tibial screw of the artificial cruciate ligament according to the present invention;
- Figs. 2 and 2A are respectively a longitudinal section and a rear view of a femoral screw of the artificial cruciate ligament according to the present invention;
- Figs. 3, 3A and 3B are respectively a side view, a front view and a rear view of a spring bushing of the artificial cruciate ligament according to the present invention;
- Figs. 4 and 4A are respectively a side view and a rear view of a pin of the artificial cruciate ligament according to the present invention;
- Figs. 5 and 5A are respectively a side view and a rear view of a bolt of the artificial cruciate ligament according to the present invention;
- Fig. 6 is a longitudinal view of the femoral screw, of the pin and of the bolt of the artificial cruciate ligament according to the present invention;
- Fig. 7 is a side view of the device of the invention;
- Figs. 8 and 8A are respectively an axonometric view and a longitudinal section of a pre-winder of the device of Fig. 7;
- Figs. 9 and 9A are respectively an axonometric view and a longitudinal section of a mounting key of the device of Fig. 7;
- Figs. 10 and 11 are respectively an axonometric view and a side view that diagrammatically illustrate the placing of the artificial cruciate ligament according to the present invention.

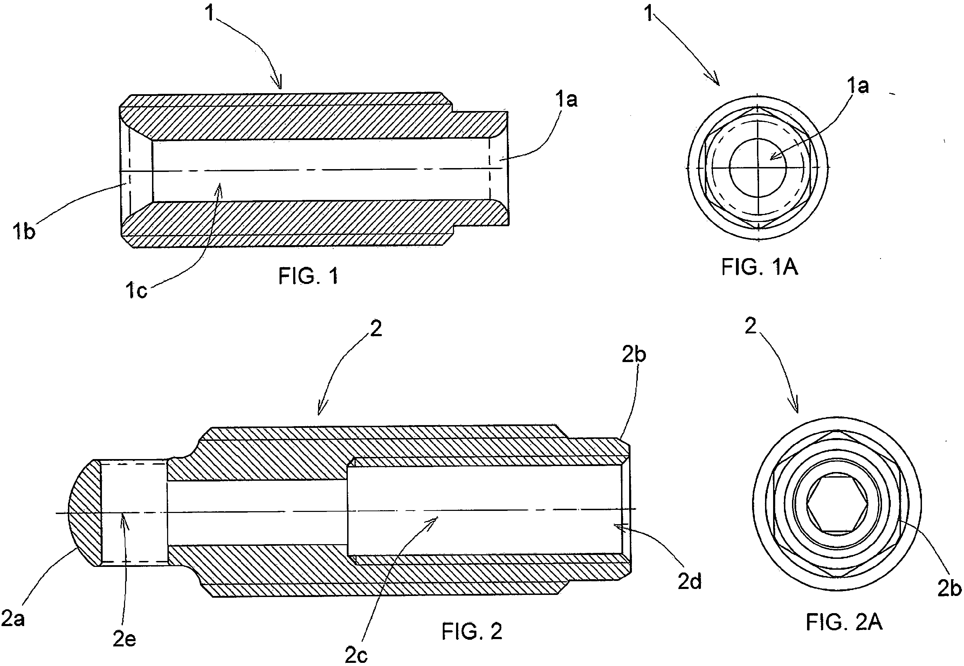

Referring to Figs. 1 and 1A, an artificial cruciate ligament (C) according to the present invention comprises a tibial screw (1) intended to be disposed inside a threaded hole obtained on a tibia (T) and comprising a through axial cavity (1c).

Moreover, the tibial screw comprises a first shaped end section (1a) with hexagonal shape, which is intended to cooperate with a tool to rotate the tibial screw (1), and a second end section (1 b) whereon a flared opening is obtained.

The artificial cruciate ligament (C) comprises a femoral screw (2), shown in Figs. 2 and 2A, intended to be disposed inside a threaded hole obtained on a femur (F).

The femoral screw (2) comprises a first closed end section (2a) and a second end section (2b) whereon an axial threaded opening (2d) is obtained. The second end section (2b) is shaped and intended to cooperate with a tool to rotate the femoral screw (2). In particular, the second end section (2b) of the femoral screw (2) has a hexagonal shape.

Moreover, the femoral screw (2) comprises a through radial hole (2e) obtained in proximal position to the first closed end section (2a) and a longitudinal blind hole (2c) which can be accessed through the axial threaded opening (2d) from one side and is closed by the first closed end section (2a) from the other side. In particular, the longitudinal blind hole (2c) and the through radial hole (2e) intersect.

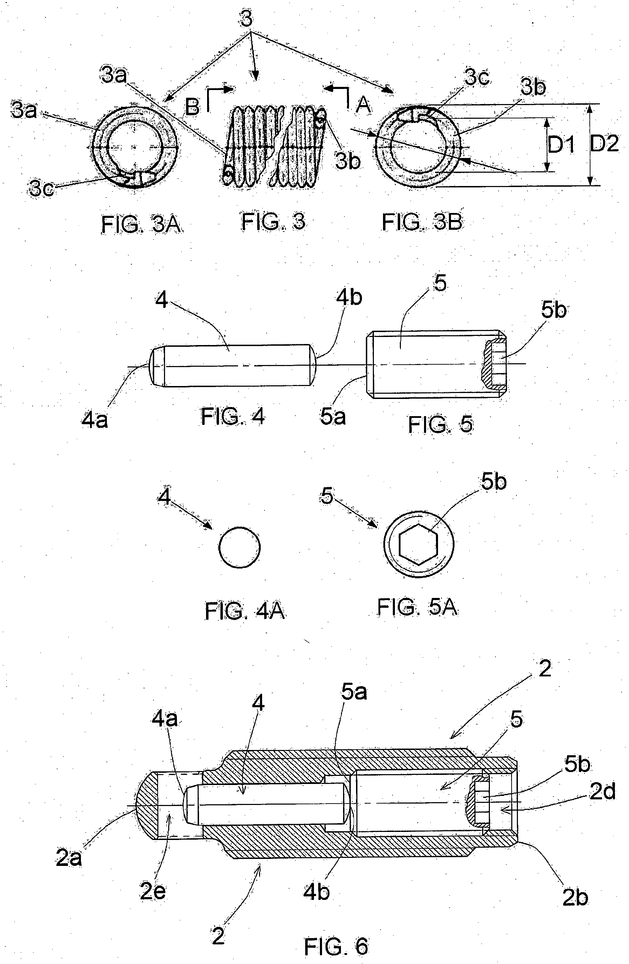

The artificial cruciate ligament (C) comprises a pin (4), shown in Figs. 4 and 4A, which is slidingly inserted in the cavity (2c) of the femoral screw (2). The pin (4) comprises a first end (4a) disposed inside the through radial hole (2e) of the femoral screw (2), and a second end (4b).

The artificial cruciate ligament (C) comprises a threaded bolt (5), shown in Figs. 5 and 5A, which is disposed in the cavity (2c) of the femoral screw (2) for locking the pin (4) in place.

The threaded bolt (5) has a first end (5a) that is stopped against the second end (4b) of the pin (4), as shown in Fig. 6, and a second end (5b) that is shaped and intended to cooperate with a tool to rotate the threaded bolt (5). In particular, the second end (5b) of the threaded bolt (5) has a hexagonal shape.

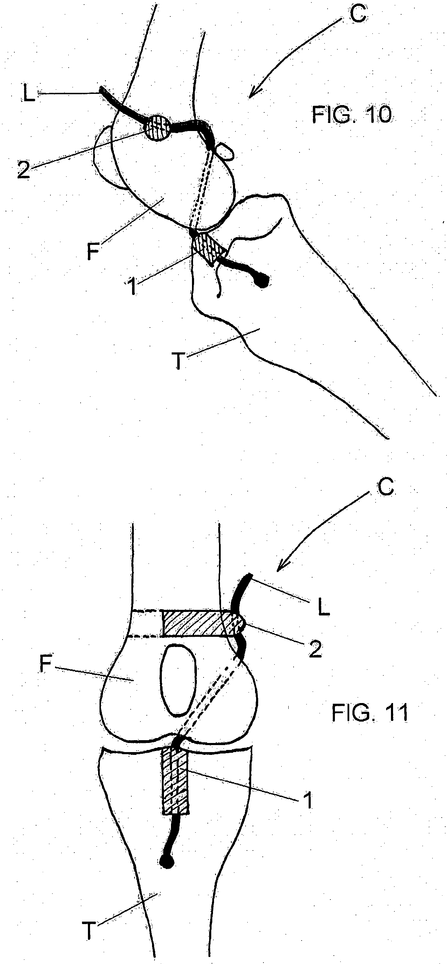

The artificial cruciate ligament (C) comprises thread-like connection means (L), as shown in Figs. 10 and 11, inserted in the axial cavity (1 c) of the tibial screw (1) and in the through radial hole (2e) of the femoral screw (2). The thread-like connection means (L) are interposed and compressed between the pin (4) and the first closed end section (2a) of the femoral screw (2).

As shown in Figs. 3, 3A and 3B, the artificial cruciate ligament (C) comprises two spring bushings (3) intended to be respectively disposed inside the threaded hole of the tibia (T) and inside the threaded hole of the femur (F), in such manner that the tibial screw (1) and the femoral screw (2) are interfaced with the spring bushings (3).

When unloaded, the spring bushings (3) have an internal diameter (D1) and an external diameter (D2) and comprise turns with a pitch (P).

Each spring bushing (3) comprises two end turns (3a, 3b). In particular, each spring bushing (3) comprises a front end turn (3a) and a rear end turn (3b). At least one end turn (3a, 3b) comprises a fixing and driving notch (3c).

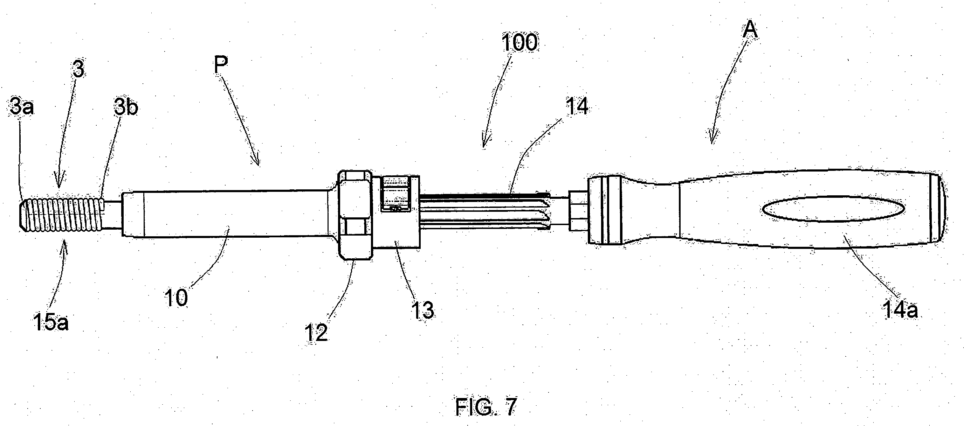

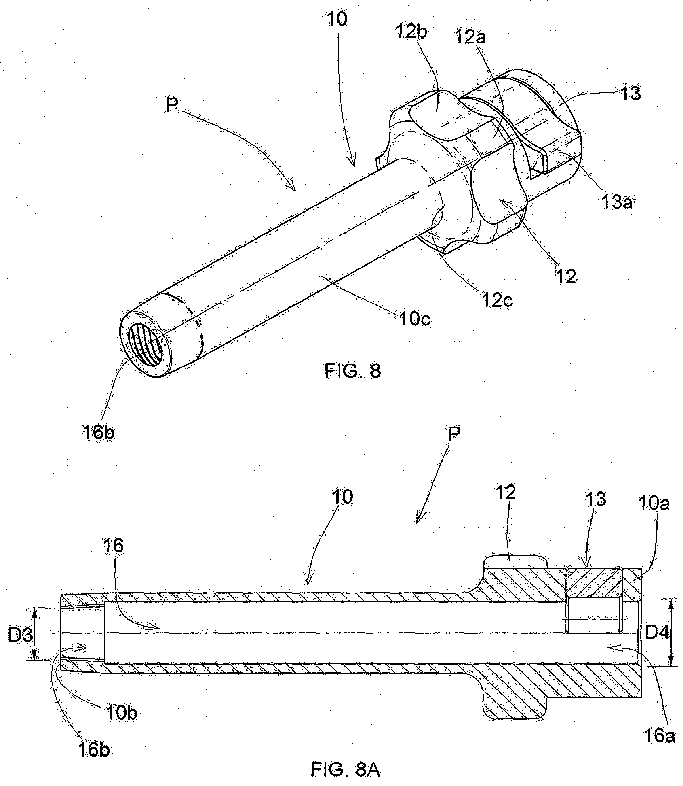

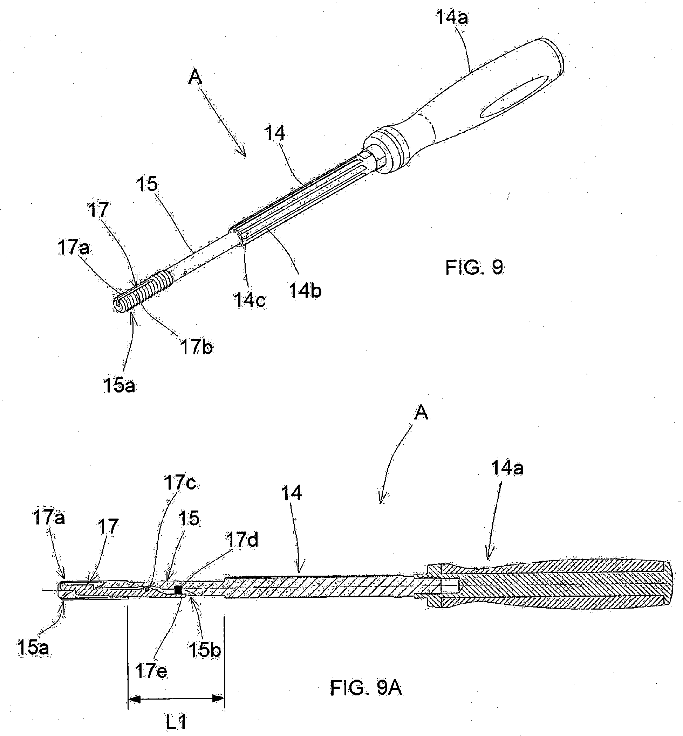

With reference to Figs. 7, 8, 8A, 9 and 9A, a mounting device according to the invention is described, being indicated with reference numeral 100. Such a device (100) allows for inserting the spring bushings (3) inside the threaded holes obtained on the tibia (T) and on the femur (F), without stressing the fragile thread of the threaded holes.

The device (100) used for placing the the spring bushings (3) of the artificial cruciate ligament (C) in position comprises a mounting key (A) configured in such manner to be engaged inside the spring bushing (3).

The mounting key (A) comprises a stem (15) with circular section provided with an opening (15b) obtained in a central portion of the stem (15) and with a threaded end section (15a) configured in such manner to be inserted in the turns of the spring bushing (3).

The threaded end section (15a) comprises an axial notch (17b) obtained in diametrally opposite position to the opening (15b) of the stem (15), and an oscillating blade (17) that is disposed inside the axial notch (17b) of the threaded end section (15a).

The oscillating blade (17) comprises a first end, which ends with a striker (17a) configured in such manner to be attached to the fixing and driving notch (3c) of the spring bushing (3), and a second end, which ends with a button (17e) that protrudes from the opening (15b) of the stem (15).

The oscillating blade (17) is hinged to the stem by means of a pin (17c) and comprises a return spring (17d) connected to the button (17e) of the oscillating blade (17) in such manner to maintain the button (17e) of the oscillating blade (17) outside the opening (15b) of the stem (15). By pushing the button (17e) of the oscillating blade (17) inside the opening (15b) of the stem (15), the oscillating blade (17) rotates around the pin (17c) and the striker (17a) is retracted in the axial notch (17b) of the threaded end section (15a). By releasing the button (17e), the return spring (17d) is unloaded, pushing the button (17e) of the oscillating blade (17) outside the opening (15b) of the stem (15). The oscillating blade (17) rotates around the pin (17c) and the striker (17a) comes out and protrudes from the notch (17b) of the threaded end section (15a).

The mounting key (A) comprises a screwing handle (14a) made of non-slip plastic material. The screwing handle (14a) is intended to be held by the user.

The mounting key (A) comprises a bar (14) that connects the screwing handle (14a) to the stem (15) and comprises ribs (14b) and grooves (14c). The ribs (14b) and the grooves (14c) of the bar (14) are at least eight.

The bar (14) and the threaded end section (15a) of the stem (15) are situated at a mutual distance (L1) of at least 25 mm, in such manner to guarantee a better visibility of the threaded end section (15) during the positioning of the spring bushings (3).

Moreover, the device (100) comprises a pre-winder (P) comprising a tubular rod (10) with an axial cavity (16) configured in such manner to house the mounting key (A).

The axial cavity (16) comprises an inlet (16a) to let the mounting key (A) enter the axial cavity (16) and an outlet (16b) to let the mounting key (A) come out from the axial cavity (16).

The diameter (D4) of the inlet (16a) is higher than the external diameter (D2) of the spring bushing (3) and the diameter (D3) of the outlet (16b) is slightly lower than the external diameter (D2) of the spring bushing (3). In fact, the external diameter (D2) of the spring bushing (3) is 10 mm, whereas the diameter (D3) of the outlet (16b) of the axial cavity (16) is lower than 10 mm, preferably 9.2 mm.

In particular, in proximal position to the outlet (16b), the axial cavity (16) of the pre-winder (P) has a conical axial section, with decreasing diameter towards the outlet (16b) until it reaches a diameter lower than 10 mm.

The outlet (16b) is threaded with the same pitch as the pitch (P) of the turns of the spring bushings (3).

The tubular rod (10) of the pre-winder (P) comprises a cylindrical wall (10c) laterally defined by a first side wall (10a) whereon the inlet (16a) of the axial cavity (16) is obtained and by a second side wall (10b) whereon the outlet (16b) of the axial cavity (16) is obtained.

The second side wall (10b) has a diameter of 13 mm, in such way to easily insert the second side wall (10b) inside the threaded holes obtained on the tibia (T) and on the femur (F).

Moreover, the pre-winder (P) comprises a holding collar (12) comprising a central hole (12a) wherein the tubular rod (10) is inserted. The holding collar (12) of the pre-winder (P) comprises ergonomic ribs (12a) and grooves (12b) intended to favor the user's finger grip.

The device (100) comprises a stopper (13) that is disposed in the inlet (16a) of the axial cavity (16) and cooperates with the bar (14) of the mounting key (A), locking the rotation of the mounting key (A) in anticlockwise direction.

The stopper (13) comprises a tooth (not shown in the attached figures) configured in such manner to be disposed in one of the grooves (14c) of the bar (14) and to stop against a rib (14b) of the bar (14) to lock the rotation of the mounting key (A) in anticlockwise direction.

The stopper (13) comprises a button (13a) to raise the tooth of the stopper (13) allowing the mounting key (A) to rotate in anticlockwise direction inside the axial cavity (16).

According to a first embodiment, the cylindrical wall (10c) comprised a loading window obtained in proximal position to the outlet (16b) of the axial cavity (16) in order to insert the spring bushing (3) in the axial cavity (16) along a radial direction.

Such an embodiment was impaired by the fact that the insertion of the spring bushing (3) in the threaded hole obtained on the tibia (T) was a difficult and very delicate operation. In fact, exactly during this step, the cylindrical wall (10c) is intended to come in contact with the tissues of a femoral joint; this implies that, having an opening defined by a perimeter edge (with more or less sharp angles), the loading window obtained on said cylindrical wall (10c) may damage the tissue because of rubbing against said perimeter edge; in fact, it must be noted that the tissues are so delicate that they can remain intact only if the wall rubbing on them is perfectly smooth and uninterrupted.

Consequently, according to a preferred embodiment, the cylindrical wall (10c) is closed, i.e. is not provided with windows or openings, and the spring bushing (3) is inserted from the inlet (16a) of the axial cavity (16) together with the mounting key (A).

As shown in Figs. 10 and 11, the method used for placing the artificial cruciate ligament (C) in position provides for drilling the blind threaded hole in the tibia (T) and the through threaded hole in the femur (F).

Successively, the spring bushings (3) are inserted in the threaded holes obtained on the tibia (T) and on the femur (F).

During the insertion of the spring bushings (3) in the threaded holes obtained on the tibia (T) and on the femur (F), the threaded end section (15a) of the mounting key (A) is screwed in the turns of the spring bushing (3), starting from the rear end turn (3b) until the fixing and driving notch (3c) of the front end turn (3a) of the spring bushing (3) is attached to the striker (17a) of the oscillating blade (17).

The mounting key (A) is inserted in the axial cavity (16) through the inlet (16a) and is screwed in a thread of the outlet (16b) of the axial cavity (16). Such screwing causes a radial contraction and an axial elongation of the spring bushing (3). During such screwing, thanks to the suitable dimensioning of the mounting key (A), the bar (14) is situated in correspondence of the stopper (13) in such manner to cooperate with the stopper (13).

The provision of the stopper (13) is motivated by the need to maintain the deformation impressed to the spring bushing (3) for the time that is needed to insert it completely into the threaded hole obtained on the tibia (T) or on the femur (F). Evidently, if such a recovery occurred earlier, when the spring bushing (3) is partially inserted in the threaded hole, the sudden radial expansion and axial compressing of the turns would cause a traumatic impact between the bone thread and the spring turns, probably breaking some of the threads.

The provision of the stopper (13) guarantees that, if the grip on the screwing handle (14a) is accidentally lost, the stem (15) will not rotate in anticlockwise direction because the bar (14) remains permanently engaged with the tooth of the stopper (13).

Moreover, such a stopper (13) can be released by means of the button (13a) used to raise the tooth of the stopper (13). Therefore, if it is necessary to unscrew the mounting key (A), the button (13a) can be pressed and the mounting key (A) can be rotated in anticlockwise direction.

The outlet (16b) of the axial cavity (16) of the tubular rod (10) is positioned in correspondence of the threaded hole obtained on the tibia (T) or on the femur (F).

The mounting key (A) is screwed in clockwise direction, in such manner to let the spring bushing (3) and the threaded end section (15a) come out from the outlet (16b) of the axial cavity (16). The penetration of the spring bushing (3) and of the threaded end section (15a) in the threaded hole will take place as long as the spring bushing (3) is in a condition of radial contraction and axial elongation, being prevented from interfering with the bone thread of the tibia (T) or of the femur (F).

The spring bushing (3) is completely inserted in the threaded hole obtained on the tibia (T) or on the femur (F). When the rear turn (3b) of the spring bushing (3) loses its coupling with the outlet (16b) of the axial cavity (16), the elastic return of the spring bushing (3) is spontaneously generated, and the turns of the spring bushing (3) recover their initial diameter and length, thus exactly matching with the thread of the threaded holes.

Then, the button (17e) of the oscillating blade (17) must be pushed in the opening (15b) of the stem (15) in order to release the fixing and driving notch (3c) of the spring bushing (3) from the striker (17a) of the oscillating blade (17).

The mounting key (A) is rotated in anticlockwise direction in order to release the threaded end section (15a) of the spring bushing (3). During such a rotation, the striker (17a) remains inside the axial notch (17b) of the threaded end section (15a).

When the threaded end section (15) of the stem (15) rotates and comes out of the spring bushing (3), the striker (17a) of the oscillating blade (17) automatically protrudes from the axial notch (17b).

Successively, the thread-like connection means (L) are prepared and a knot is made on a first end of the thread-like connection means (L).

A second end of the thread-like connection means (L) is inserted in the axial cavity (1c) of the tibial screw (1), in such manner that the knot is engaged against the second end section (1 b) of the tibial screw (1).

The tibial screw (1) is screwed in the spring bushing (3) disposed in the threaded hole obtained on the tibia (T) in such manner that the knot of the thread-like connection means (L) remains inside the tibia (T). The tibial screw (1) is disposed in the threaded hole of the tibia (T) with the first shaped end section (1 a) facing outwards and the second end section (1 b) in contact with the tibia (T).

The femoral screw (2) is screwed in the spring bushing (3) disposed in the threaded hole obtained in the femur (F), leaving the first closed end section (2a) of the femoral screw (2) outside the threaded hole.

A second end of said thread-like connection means (L) is inserted in the radial through hole (2e) of the femoral screw (2).

The pin (4) and the threaded bolt (5) are inserted in the longitudinal blind hole (2c) of the femoral screw (2) and the threaded bolt (5) is screwed in such manner to push the pin (4) against the thread-like connection means (L) to lock the thread-like connection means (L) between the first closed end section (2a) of the femoral screw (2) and the pin (4).

The advantages of the device (100) according to the present invention are evident, because it makes it possible to insert the spring bushings (3) inside the threaded holes obtained on the tibia (T) and on the femur (F), without touching the thread of the threaded holes.

In spite of having described an oscillating blade (17) with a striker (17a) which is engaged into or released from the fixing and driving notch (3c) of the spring bushing (3) by means of the button (17e) of the oscillating blade (17), it is possible to provide a fixing and driving notch (3c) with inclined connecting sections whereon the striker (17a) slides, being progressively and automatically disposed in extracted or retracted position with respect to the axial notch (17b), without using the button (17e).

## Claims

1. Artificial cruciate ligament (C) for a knee, comprising:
- a tibial screw (1) intended to be disposed inside a threaded hole obtained on a tibia (T) and comprising a through axial cavity (1 c); said tibial screw (1) comprising a first shaped end section (1 a) intended to cooperate with a tool to rotate the tibial screw (1);
- a femoral screw (2) intended to be disposed inside a threaded hole obtained on a femur (F); said femoral screw (2) comprising a first closed end section (2a) and a second end section (2b) whereon an axial threaded opening (2d) is obtained; said femoral screw (2) comprising a longitudinal blind hole (2c) that can be accessed through the axial threaded opening (2d) from one side and is closed by said closed end section (2a) from the other side; said femoral screw (2) comprising a through radial hole (2e) in proximal position to said first closed end section (2a) that intersects with said longitudinal blind hole (2c); said second end section (2b) being shaped and intended to cooperate with a tool to rotate the femoral screw (2);
- a pin (4) slidingly inserted in the cavity (2c) of the femoral screw (2);
- a threaded bolt (5) disposed in the cavity (2c) of the femoral screw (2) and having a first end (5a) stopped against the pin (4) and a second shaped end (5b) intended to cooperate with a tool to rotate the threaded bolt (5);
- thread-like connection means (L) inserted in the axial cavity (1c) of the tibial screw and in the through radial hole (2e) of the femoral screw (2);said thread-like connecting means (L) being interposed and compressed between the pin (4) and the first closed end section (2a) of the femoral screw (2);
- two spring bushings (3) intended to be respectively disposed inside the threaded hole of the tibia (T) and inside the threaded hole of the femur (F); said tibial screw (1) and said femoral screw (2) being interfaced with said spring bushings (3); said spring bushings (3) having an interval diameter (D1) and an external diameter (D2) and comprising turns with a pitch (P).

2. The artificial cruciate ligament (C) of claim 1, wherein each spring bushing (3) comprises an end turn (3a, 3b) provided with a fixing and driving notch (3c).

3. The artificial cruciate ligament (C) of claim 1 or 2, wherein the first shaped end section (1a) of the tibial screw (1) and the second end section (2b) of the femoral screw (2) have a hexagonal shape; said tibial screw (1) comprising a second end section (1 b) whereon a flared opening is obtained.

4. Device (100) for placing in position the spring bushings (3) of the artificial cruciate ligament (C) described in any one of the preceding claims; said device (100) comprising:
a) a mounting key (A) configured in such manner to be engaged inside said spring bushing (3); said mounting key (A) comprising:
- a stem (15) with circular section comprising a threaded end section (15a) configured in such manner to be inserted in the turns of the spring bushing (3); said threaded end section (15a) comprising an axial notch (17b) and an oscillating blade (17) that is disposed inside said axial notch (17b); said oscillating blade (17) ending with a striker (17a) configured in such manner to be attached to the fixing and driving notch of the spring bushing (3);
- a screwing handle (14a) intended to be held by a user;
- a bar (14) connecting said screwing handle (14a) to the stem (15); said bar (14) comprising ribs (14b) and grooves (14c).
b) a pre-winder (P) comprising:
- a tubular rod (10) provided with an axial cavity (16) configured in such manner to house the mounting key (A); said axial cavity (16) comprising an inlet (16a) to let the mounting key (A) enter the axial cavity (16) and an outlet (16b) to let the mounting key (A) come out from the axial cavity (16); said inlet (16a) having a higher diameter than the external diameter (D2) of the spring bushing (3); said outlet (16b) having a slightly lower diameter (D3) than the external diameter (D2) of the spring bushing (3); said outlet (16b) being threaded with the same pitch as the turns of the spring bushing (3);
- a holding collar (12) comprising a central hole (12a) wherein the tubular rod (10) is inserted;
c) a stopper (13) that cooperates with the bar (14) of the mounting key (A), locking the rotation of the mounting key (A) in anticlockwise direction.

5. The device (100) of claim 4, wherein the screwing handle (14a) of the mounting key (A) is made of non-slip plastic material.

6. The device (100) of claim 4 or 5, wherein the bar (14) and the threaded end section (15a) of the stem (15) are positioned at a mutual distance (L1) of at least 25 mm.

7. The device (100) of any one of claims 4 to 6, wherein the ribs (14b) of the bar (14) are at least eight and the grooves (14c) of the bar (14) are at least eight.

8. The device (100) of any one of claims 4 to 7, wherein the tubular rod (10) of the pre-winder (P) comprises a cylindrical wall (10c) laterally defined by a first side wall (10a) whereon the inlet (16a) of the axial cavity (16) is obtained and by a second side wall (10b) whereon the outlet (16b) of the axial cavity (16) is obtained.

9. The device (100) of any one of claims 4 to 8, wherein said axial cavity (16) of the pre-winder (P), in proximal position to its outlet (16b), has a conical axial section, with decreasing diameter towards the outlet (16b).

10. The device (100) of any one of claims 4 to 9, wherein the outlet (16b) of the axial cavity (16) of the pre-winder (P) has a diameter (D3) lower than 10 mm.

11. The device (100) of any one of claims 4 to 10, wherein the stopper (13) comprises a tooth configured in such manner to be disposed in one of the grooves (14c) of the bar (14) and to stop against a rib (14b) of the bar (14) to lock the rotation of the mounting key (A) in anticlockwise direction.

12. The device (100) of claim 11, wherein said stopper (13) comprises a button (13a) to raise the tooth of the stopper (13), thus allowing the mounting key (A) to rotate in anticlockwise direction inside the axial cavity (16).

13. The device (100) of any one of claims 4 to 12, wherein said holding collar (12) of the pre-winder (P) comprises ergonomic ribs (12a) and grooves (12b) intended to facilitate the user's finger grip.

14. Method for placing in position the artificial cruciate ligament (C) as disclosed in any one of the preceding claims, providing for:
- drilling the blind threaded hole in the tibia (T);
- drilling the through threaded hole in the femur (F);
- inserting the spring bushings (3) in the threaded holes obtained on the tibia (T) and on the femur (F);
- arranging the thread-like connecting means (L);
- making a knot on a first end of said thread-like connecting means (L);
- inserting a second end of said thread-like connecting means (L) in the axial cavity (1c) of the tibial screw (1), in such manner that the knot is engaged against said tibial screw (1);
- inserting the tibial screw (1) in the threaded hole obtained on the tibia (T) in such manner that the knot of the thread-like connection means (L) remains inside the tibia (T);
- inserting the femoral screw (2) in the threaded hole obtained on the femur (F), leaving the first end section (2a) of the femoral screw (2) outside the threaded hole;
- inserting the second end of said thread-like connecting means (L) in the through radial hole (2e) disposed on the first end section (2a) of the femoral screw (2);
- screwing the threaded bolt (5) in such manner to push the pin (4) against the thread-like connection means (L) to lock the thread-like connection means (L).

15. The method for placing in position the artificial cruciate ligament (C) of claim 14, when depending on claims 4 to 13, wherein the insertion step of the spring bushings (3) in the threaded holes obtained on the tibia (T) and on the femur (F) provides for:
- screwing the threaded end section (15a) of the mounting key (A) in the turns of the spring bushing (3);
- attaching the fixing and driving notch (3c) of the spring bushing (3) to the striker (17a) of the oscillating blade (17);
- inserting the mounting key (A) in the axial cavity (16) through the inlet (16a);
- screwing the mounting key (A) in a thread of the outlet (16b) of the axial cavity (16); said screwing causing a radial contraction and an axial elongation of said spring bushing (3);
- positioning the outlet (16b) of the axial cavity (16) of the tubular rod (10) in correspondence of the threaded hole obtained on the tibia (T) or on the femur (F).
- screwing the mounting key (A) in such manner to let the spring bushing (3) and the threaded end section (15a) come out from the outlet (16b), completely inserting the spring bushing (3) in the threaded hole obtained on the tibia (T) or on the femur (F);
- rotating the mounting key (A) in anticlockwise direction in order to release the threaded end section (15a) from the spring bushing (3).
